# EUROPÄISCHE PATENTANMELDUNG

(11) **EP 4 385 562 A1**
(43) Veröffentlichungstag der Anmeldung: **19.06.2024**
(21) Anmeldenummer: 22214198.8
(22) Anmeldetag: 16.12.2022
(51) Int. Cl.: A61N 5/10

(54) **VERFAHREN ZUM ERZEUGEN VON STEUERDATEN FÜR EINE BESTRAHLUNGSVORRICHTUNG, COMPUTERPROGRAMMPRODUKT, COMPUTERLESBARES SPEICHERMEDIUM, ELEKTRONISCHE RECHENEINRICHTUNG SOWIE BESTRAHLUNGSVORRICHTUNG**

(71) Anmelder: Siemens Healthineers AG, 91301 Forchheim (DE)
(72) Erfinder: Dr. Fenchel, Matthias, 91054 Erlangen (DE); Dr. Vahle, Thomas, 90409 Nürnberg (DE); Wehrl, Hans, 91052 Erlangen (DE)
(74) Vertreter: Siemens Healthineers Patent Attorneys

(57) **Zusammenfassung**

Die Erfindung betrifft ein Verfahren zum Erzeugen von Steuerdaten (18) für eine Bestrahlungsvorrichtung (12) für einen Patienten (20) mittels einer elektronischen Recheneinrichtung (16) der Bestrahlungsvorrichtung (12), mit den Schritten:
- Bereitstellen eines Bewegungsmodells (28a, 28b) des Patienten (20) für eine Bestrahlung (22) in der Bestrahlungsvorrichtung (12); und
- Erzeugen von Steuerdaten (18) für die Bestrahlung (22) des Patienten (20) in Abhängigkeit von zumindest einer Patienteninformation und in Abhängigkeit von dem Bewegungsmodell (28a, 28b). Ferner betrifft die Erfindung ein Computerprogrammprodukt, ein computerlesbares Speichermedium, eine elektronische Recheneinrichtung sowie eine Behandlungvorrichtung (12).

## Beschreibung

Die Erfindung betrifft ein Verfahren zum Erzeugen von Steuerdaten für eine Bestrahlungsvorrichtung für Patienten mittels einer elektronischen Recheneinrichtung der Bestrahlungsvorrichtung. Ferner betrifft die Erfindung ein Computerprogrammprodukt, ein computerlesbares Speichermedium, eine elektronische Recheneinrichtung sowie eine Bestrahlungsvorrichtung.

Die Strahlentherapie ist eine der am häufigsten eingesetzten Methoden zur Krebsbehandlung. In vielen Fällen wird die externe Strahlentherapie eingesetzt, welche auch bekannt als Teletherapie oder perkutane Strahlentherapie ist. In der Regel wird der Patient in der Nähe des Linearbeschleunigers (Linac) platziert, und das Zielvolumen, zum Beispiel der Tumor, wird einige Minuten lang fraktioniert bestrahlt, das heißt diese Behandlungssitzungen wiederholen sich über einen Zeitraum von Tagen beziehungsweise Wochen. Während einer Behandlungssitzung, und auch dazwischen, kann sich das Gewebe im Körper des Patienten relativ zum Referenzpunkt bewegen, der für die Strahlentherapieplanung verwendet wurde. Daher kann sich das geplante Zielvolumen außerhalb des Strahlenbündels bewegen oder gesundes Gewebe kann während der Behandlung in das Strahlenbündel hineinwandern. Beide Effekte wirken sich negativ auf den Behandlungserfolg aus, weil entweder die Dosis für das Zielgewebe zu niedrig ist oder gesundes Gewebe bestrahlt und in der Folge geschädigt wird, was zu Nebenwirkungen der Strahlentherapie führt. Atembewegungen stellen dabei eine der größten Herausforderungen dar, insbesondere im Bauch- und Thoraxbereich, aber auch andere Bewegungsarten sind möglich. Trotz vieler Ansätze bleibt die Bewegung während der Behandlung eine große Herausforderung bei der Strahlentherapie, die auch das Potenzial von Techniken wie der intensitätsmodulierten Strahlentherapie (IMRD) beeinträchtigt.

Aufgabe der vorliegenden Erfindung ist es ein Verfahren, ein Computerprogrammprodukt, ein computerlesbares Speichermedium, eine elektronische Recheneinrichtung sowie eine Behandlungsvorrichtung zu schaffen, mittels welchen eine verbesserte Bestrahlung eines Patienten ermöglicht wird.

Diese Aufgabe wird durch ein Verfahren, ein Computerprogrammprodukt, ein computerlesbares Speichermedium, eine elektronische Recheneinrichtung sowie eine Bestrahlungsvorrichtung gemäß den unabhängigen Patentansprüchen gelöst. Vorteilhafte Ausgestaltungsformen sind in den Unteransprüchen angegeben.

Ein Aspekt der Erfindung betrifft ein Verfahren zum Erzeugen von Steuerdaten für eine Bestrahlungsvorrichtung für Patienten mittels einer elektronischen Recheneinrichtung der Bestrahlungsvorrichtung. Es erfolgt das Bereitstellen eines Bewegungsmodells des Patienten für eine Behandlung in der Bestrahlungsvorrichtung und das Erzeugen von Steuerdaten für eine Bestrahlung des Patienten in Abhängigkeit von zumindest einer Patienteninformation und in Abhängigkeit von dem Bewegungsmodell.

Insbesondere ist somit ein Verfahren vorgestellt, um die Bewegung auch während einer Bestrahlung, und auch in einer weiteren Ausführungsform auch zwischen den entsprechenden Sitzungen entsprechend vorherzusagen. Das Problem entsteht dabei nicht nur in der Linac-basierten Strahlentherapie, sondern auch bei anderen Arten der Strahlentherapie, zum Beispiel mit einem Gamma Knife. Der Fortschritt der IMRT, die noch genauer ist, konzentriert sich noch mehr auf die Herausforderungen der Patientenbewegung während und zwischen den entsprechenden Therapiesitzungen.

Insbesondere kann somit eine Bewegungskompensation auf Grundlage eines Bewegungsmodells beziehungsweise auch eines sogenannten digitalen Zwillings des Patienten während der Strahlentherapie durchgeführt werden. Insbesondere wird dabei ein Bewegungsmodell auf einen zeitaufgelösten Scan, beispielsweise auf einem Magnetresonanzscan, und einem während des Magnetresonanzscans erfassten Atmungsmodells abgeleitet. Dieses Bewegungsmodell wird auf die Strahlentherapie-Behandlung übertragen, indem das Atmungssignal während der Bestrahlung erfasst und das vom Scan abgeleitete Bewegungsmodell daran angepasst wird. Dies liefert ein Bewegungsmodell, beispielsweise der inneren Organe und der Zielzone für die Bestrahlung, dass eine bewegungskorrigierte Steuerung des Bestrahlungssystems beziehungsweise der Bestrahlungsvorrichtung, zum Beispiel des Linac, ermöglicht. Andere Informationsquellen, zum Beispiel im Behandlungsröntgenbild, können zusätzlich ergänzt werden, um die Genauigkeit des Modells und die Anpassung an die jeweilige Bewegungsphase zu verbessern. Idealerweise wird der Patient ohne einen Positionswechsel auf einem Transferbett, zum Beispiel ähnlich einer Strahlentherapie-Trage beziehungsweise -Bett transportiert, wobei beispielsweise ein Beatmungsgurt als Signalgeber an Ort und Stelle verbleibt.

Insbesondere entspricht somit die Bestrahlung selbst der Strahlentherapie. Als Patienteninformationen können beispielsweise eine Verortung eines Tumors, entsprechende Gewebewerte, Blutwerte oder dergleichen herangezogen werden. Somit kann die Behandlung zuverlässig realisiert werden, da auf Basis des Verfahrens erzeugte Steuerdaten entsprechend eine verbesserte Behandlung zulassen.

Das Bewegungsmodell basiert dabei insbesondere auf einem Bildgebungsverfahren für den zu behandelnden Bereich. Dieses Bildgebungsverfahren kann auch als Scan bezeichnet werden.

Gemäß einer vorteilhaften Ausgestaltungsform wird das Bewegungsmodell auf Basis einer außerhalb der Bestrahlungsvorrichtung erfassten Bewegung des Patienten zeitlich vor der Bestrahlung bestimmt. Insbesondere wird somit das Bewegungsmodell bereits zeitlich vor der eigentlichen Bestrahlung bestimmt, beispielsweise in einer Magnetresonanzanlage, und bereitgestellt, sodass eine wesentliche echtzeitfähige Bestrahlung auf Basis des Bewegungsmodells durchgeführt werden kann.

Ferner hat es sich als vorteilhaft erwiesen, wenn auf Basis einer mittels einer Magnetresonanzanlage bestimmten Bewegung des Patienten in der Magnetresonanzanlage das Bewegungsmodell erzeugt wird. Insbesondere die Magnetresonanzanlage bietet sich dabei an, da ein hochauflösendes Bildgebungsverfahren/Scan hierbei benutzt wird. Insbesondere können beispielsweise entsprechende Atembewegungen und dabei entstehende Bewegungen innerhalb des Körpers, beispielsweise der Organe, entsprechend aufgezeichnet werden und eine entsprechende Bewegung derselben vorhergesagt werden.

Weiterhin kann vorgesehen sein, dass eine Position des Patienten in der Magnetresonanzanlage und eine Position des Patienten in der Bestrahlungsvorrichtung zumindest im Wesentlichen gleich eingestellt werden. Insbesondere kann beispielsweise vorgesehen sein, dass sich die Magnetresonanzanlage in der Nähe, beispielsweise im gleichen Raum, mit der Bestrahlungsvorrichtung selbst befindet. Insbesondere kann die Magnetresonanzanlage mit der Bestrahlungsvorrichtung ein entsprechendes Bestrahlungssystem ausbilden. Es kann dann eine entsprechende Liege/Bett/Trage in der Magnetresonanzanlage verwendet werden, auf welcher der Patient positioniert wird und das entsprechende Bewegungsmodell erzeugt wird. Auf der gleichen Liege und in der gleichen Position kann dann wiederum der Patient direkt in die Bestrahlungsvorrichtung eingefahren werden, um beispielsweise die Bestrahlung auf Basis der erzeugten Bestrahlungssteuerdaten durchgeführt werden. Somit kann hochpräzise eine Bestrahlung durchgeführt werden.

Ferner hat es sich als vorteilhaft erwiesen, wenn eine Atmung als die Bewegung des Patienten im Bewegungsmodell berücksichtigt wird. Insbesondere die Atmung hat entsprechende Auswirkungen auf die Organe im Thorax. Die Atmung kann dabei während der Bestrahlung nicht abgestellt werden, sodass eine Bewegung der Organe hierbei immer zu verzeichnet ist. Auf Basis der Atmung kann nun ein Vorhersagemodell für die Atmung und insbesondere für die Bewegung der Organe erzeugt werden, sodass die Steuerdaten entsprechend an die Atmung angepasst werden können.

Eine weitere vorteilhafte Ausgestaltungsform sieht vor, dass eine Bewegung von Organen des Patienten mittels des Bewegungsmodells vorhergesagt wird. Insbesondere kann somit die Bewegung der Organe, insbesondere im Thoraxbereich, beispielsweise auf Basis der Atmung, vorhergesagt werden, sodass eine zuverlässige Bestrahlung realisiert werden kann.

Eine weitere vorteilhafte Ausgestaltungsform sieht vor, dass mittels der Steuerdaten eine Intensität der Bestrahlung und/oder eine Position der Bestrahlung und/oder ein Zeitverlauf der Bestrahlung eingestellt werden. Dadurch ist es möglich, dass eine zuverlässige Bestrahlung durchgeführt werden kann, da beispielswiese die Intensität, mit anderen Worten eine Bestrahlungsstärke beziehungsweise Position entsprechend angepasst werden kann. Insbesondere die Bewegung der Organe kann somit berücksichtigt werden. Des Weiteren kann auch ein Zeitverlauf der Bestrahlung eingestellt werden, beispielsweise kann in Abhängigkeit eines Zeitverlaufs der Atmung die entsprechende Bestrahlung angepasst werden, sodass lediglich eine sogenannte Bestrahlungszone bestrahlt wird.

Ferner hat es sich als vorteilhaft erwiesen, wenn eine Bestrahlungszone für die Bestrahlung vorgegeben wird und das Bewegungsmodell zumindest für die Bestrahlungszone mittels der elektronischen Recheneinrichtung erzeugt wird. Bei der Bestrahlungszone handelt es sich insbesondere um eine Zone des Patienten, auf welchen die Strahlentherapie, mit anderen Worten die Bestrahlung selbst, angewendet wird. Es ist nun vorteilhaft, wenn zumindest für die Bestrahlungszone die Bewegung, beispielsweise der Organe, vorhergesagt wird, sodass eine vorteilhafte Bestrahlung der Bestrahlungszone durchgeführt werden kann.

Eine weitere vorteilhafte Ausgestaltungsform sieht vor, dass während einer Bestrahlung des Patienten eine aktuelle Atmung des Patienten erfasst wird und auf Basis der aktuellen Atmung die Steuerdaten erzeugt werden. Beispielsweise kann die Atmung mittels eines Atemgurtes und/oder mittels einer Kamera während der Bestrahlung erfasst werden. Somit ist es ermöglicht, dass die aktuelle Atmung ebenfalls mitberücksichtigt wird, und mit in das Bewegungsmodell einfließt, wodurch eine noch genauere Vorhersage der Bewegung, insbesondere der Organe, realisiert werden kann. Somit kann eine äußerst effiziente Strahlentherapie bereitgestellt werden.

Eine weitere vorteilhafte Ausgestaltungsform sieht vor, dass ein Atemsignal für den Patienten während der Bestrahlung zum Nachatmen vorgegeben wird. Beispielsweise kann eine nachzuatmende Atemkurve optisch angezeigt werden, sodass der Patient entsprechend dem Atemsignal nachatmen kann. Somit ist es noch effizienter ermöglicht, die Vorhersage durchzuführen, da auf Basis des vorgegebenen Atemsignals die Bewegung der Organe noch präziser vorhergesagt werden kann.

Bei dem vorgestellten Verfahren handelt es sich insbesondere um ein computer-implementiertes Verfahren. Daher betrifft ein weiterer Aspekt der Erfindung ein Computerprogrammprodukt mit Programmcodemitteln, welche eine elektronische Recheneinrichtung zu veranlassen, wenn die Programmcodemittel von der elektronischen Recheneinrichtung abgearbeitet werden, ein Verfahren nach dem vorhergehenden Aspekt durchzuführen. Das Computerprogrammprodukt kann auch als Computerprogramm bezeichnet werden.

Ferner betrifft die Erfindung auch ein computerlesbares Speichermedium mit zumindest dem Computerprogrammprodukt nach dem vorhergehenden Aspekt.

Ein weiterer Aspekt der Erfindung betrifft eine elektronische Recheneinrichtung zum Erzeugen von Steuerdaten für eine Bestrahlungsvorrichtung für einen Patienten, wobei die elektronische Recheneinrichtung zum Durchführen eines Verfahrens nach dem vorhergehenden Aspekt ausgebildet ist. Insbesondere wird das Verfahren mittels der elektronischen Recheneinrichtung durchgeführt.

Die elektronische Recheneinrichtung weist beispielsweise Prozessoren, Schaltkreise, insbesondere integrierte Schaltkreise, sowie weitere elektronische Bauteile auf, um entsprechende Verfahrensschritte durchführen zu können.

Ferner betrifft die Erfindung auch eine Bestrahlungsvorrichtung zum Bestrahlen von einem Patienten, mit zumindest einer elektronischen Recheneinrichtung nach dem vorhergehenden Aspekt.

Weiterhin betrifft die Erfindung auch ein Bestrahlungssystem mit zumindest der Bestrahlungsvorrichtung und mit einer Magnetresonanzanlage. Bevorzugt können die Bestrahlungsvorrichtung und die Magnetresonanzanlage in einem gemeinsamen Raum ausgebildet sein.

Unabhängig vom grammatikalischen Geschlecht eines bestimmten Begriffes sind Personen mit männlicher, weiblicher oder anderer Geschlechteridentität mit umfasst.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Weitere Merkmale und Vorteile sind der folgenden Beschreibung anhand der beigefügten FIG. zu entnehmen. In der FIG. bezeichnen gleiche Bezugszeichen gleiche Merkmale und Funktionen. Die Ausführungsbeispiele dienen lediglich der Erläuterung der Erfindung und sollen diese nicht beschränken.

Die Erfindung wird nun anhand eines Ausführungsbeispiels in der FIG näher beschrieben.

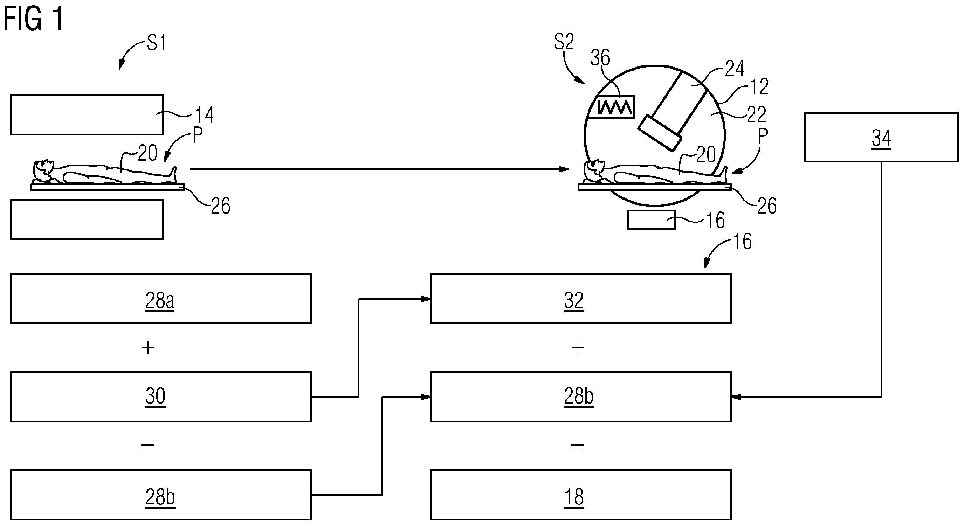

Dabei zeigt die FIG 1 ein schematisches Blockschaltbild gemäß einer Ausführungsform des Verfahrens.

FIG 1 zeigt ein schematisches Blockschaltbild einer Ausführungsform eines im Ganzen bezeichneten Bestrahlungssystems 10. Das Bestrahlungssystem 10 weist zumindest eine Bestrahlungsvorrichtung 12 sowie eine Magnetresonanzanlage 14 auf. Bevorzugt ist das Bestrahlungssystem 10 in einem eigenständigen Raum untergebracht.

Die Bestrahlungsvorrichtung 12 weist insbesondere eine elektronische Recheneinrichtung 16 auf. Die elektronische Recheneinrichtung 16 ist zum Erzeugen von Steuerdaten 18 für die Bestrahlungsvorrichtung 12 für einen Patienten 20 ausgebildet. Eine Bestrahlung 22 wird dabei insbesondere mit einer Bestrahlungseinrichtung 24 der Bestrahlungsvorrichtung 12 durchgeführt.

In dem der FIG 1 gezeigten Ausführungsbeispiel befindet sich beispielsweise der Patient 20 auf einer Trage 26, welche sich in einem ersten Schritt S1 in der Magnetresonanzanlage 14 befindet und in einem zweiten Schritt S2 wiederum in der Bestrahlungsvorrichtung 12 befindet.

Bei einem vorgestellten Verfahren zum Erzeugen der Steuerdaten 18 ist vorgesehen, dass ein Bewegungsmodell 28a, 28b des Patienten 20 für eine Behandlung in der Bestrahlungsvorrichtung 12 bereitgestellt wird und das Erzeugen der Steuerdaten 18 für eine Bestrahlung des Patienten in Abhängigkeit von zumindest einer Patienteninformation und in Abhängigkeit von dem Bewegungsmodell 28a, 28b durchgeführt wird.

Insbesondere zeigt die FIG 1, dass ein Bewegungsmodell 28a für den gesamten Patienten 20 erzeugt wird und ein Bewegungsmodell 28b wiederum lediglich für die Organe des Patienten 20 bereitgestellt wird. Zum Erzeugen des Bewegungsmodells 28b für die Organe wird insbesondere noch ein Atmungssignal 30 während des Scans, als ein Bildaufnahme des Patienten 20 in der Magnetresonanzanlage 14, berücksichtigt.

Insbesondere ist ferner gezeigt, dass eine aktuelle Atmung 32 innerhalb der Behandlungsvorrichtung 12 ebenfalls miterfasst wird und auf Basis der aktuellen Atmung 32 und dem Bewegungsmodell 28b der Organe wiederum die Steuerdaten 18 erzeugt werden. Ferner können noch weitere Informationen 34, beispielsweise Röntgenbilder, mitberücksichtigt werden.

Insbesondere zeigt die FIG 1, dass das Bewegungsmodell 28a, 28b auf Basis der außerhalb der Bestrahlungsvorrichtung 12 ausgebildeten Magnetresonanzanlage 14 erzeugt wird. Ferner zeigt die FIG 1 auch, dass eine Position P des Patienten 20 in der Magnetresonanzanlage 14 und eine Position P des Patienten 20 in der Bestrahlungsvorrichtung 12 zumindest im Wesentlichen gleich eingestellt werden.

Insbesondere kann ferner vorgesehen sein, dass mittels der Steuerdaten eine Intensität der Bestrahlung 22 und/oder eine Position der Bestrahlung 22 und/oder ein Zeitverlauf der Bestrahlung 22 eingestellt werden.

Ferner kann vorgesehen sein, dass die aktuelle Atmung 32 mittels eines Atemgurtes und/oder mittels einer Kamera während der Bestrahlung 22 und/oder eine Atmung in der Magnetresonanzanlage 14 erfasst wird.

Des Weiteren zeigt die FIG 1, dass die Bestrahlungsvorrichtung 12 auch eine Anzeigeeinrichtung 36 aufweisen kann, wobei mittels der Anzeigeeinrichtung 36 ein Atemsignal für den Patienten 20 während der Bestrahlung 22 zum Nachatmen vorgegeben wird.

Insbesondere zeigt somit die FIG 1 eine schematische Darstellung einer möglichen Ausführungsform der Bewegungskorrektur während der Bestrahlung 22, welche auch als Strahlentherapie-behandlung bezeichnet werden kann, auf der Grundlage der Bildgebung der Magnetresonanzanlage 14 und eines davon abgeleiteten Bewegungsmodells 28a, 28b. Ein Bewegungsmodell 28a wird aus einem zeitaufgelösten MR-Scan und einem während des MR-Scans erfassten Atmungssignal abgeleitet. Dieses Bewegungsmodell 28a wird wiederum auf die Organe als Bewegungsmodell 28b abgeleitet und für die Bestrahlung 22 übertragen, indem das Atmungssignal während der Behandlung erfasst und das vom Scan abgeleitete Bewegungsmodell 28a, 28b daran angepasst wird. Dies liefert beispielsweise ein Bewegungsmodell 28b der inneren Organe und der Zielzone für die Bestrahlung 22, dass eine bewegungskorrigierte Steuerung der Bestrahlungsvorrichtung 12, zum Beispiel des Linac, ermöglicht. Andere Informationsquellen, zum Beispiel im Behandlungsröntgenbild, können ergänzt werden um die Genauigkeit des Bewegungsmodells 28a, 28b und die Anpassung an die jeweilige Bewegungsphase zu verbessern. Idealerweise wird der Patient 20 ohne Positionswechsel auf einem Transferbrett, zum Beispiel ähnlich einer RT-Tischplatte, vorliegend insbesondere der Trage 26, transportiert, wobei der Beatmungsgurt als Signalgeber an Ort und Stelle verbleibt.

Ferner zeigt die FIG 1 insbesondere, dass der Patient 20 auf die Tischplatte der Bestrahlungsvorrichtung 12 gelegt wird und in der Magnetresonanzanlage 14 gescannt wird, welche sich in der Nähe der Bestrahlungsvorrichtung 12 befindet. Dies kann entweder im selben Krankenhaus, Abteilung oder sogar im selben Raum wie die Bestrahlungsvorrichtung 12 sein. Die Scansitzung dient der Untersuchung des Zielgebiets, zum Beispiel eines Tumors. Ein Atemgürtel wird dem Patienten 20 angelegt, um eine Atemkurve zu überwachen und aufzuzeichnen. Das Ausgangssignal dieses Atemgürtels wird in die Magnetresonanzanlage 14 eingespeist und verarbeitet. Gleichzeitig wird auch der Scan des Zielgebiets durchgeführt. In einem ähnlichen Ansatz wie beim sogenannten Body Compass kann somit eine Sequenz ermittelt werden, die in freier Atmung durchgeführt wird. Diese Magnetresonanz-Sequenz liefert ein bewegungsaufgelöstes vierdimensionales Bild des Zielgebietes, wobei hier auch weitere Magnetresonanz-Sequenzen, beispielsweise mit Beschleunigungstechniken oder dergleichen möglich sind. Dies ermöglicht eine Korrelation der einzelnen Bewegungszustände mit dem gleichzeitig erfassten Atmungssignal. Auf der Grundlage dieser Information, beispielsweise Organ- und Zielgebietspositionen und der mit dem Atemgurt gebildeten Atemkurve wird dann das Bewegungsmodell 28b der Organe erstellt.

Nach Abschluss des Scans wird der Patient 20 zur Bestrahlungsvorrichtung 12, zum Beispiel Linac, gebracht. Der Patient 20 wird auf dem Linac in die Behandlungsposition gebracht. Auf Grund der Trage 26 kann die Position P in der Behandlungsvorrichtung 12 mit der Position P in der Magnetresonanzanlage 14 im Wesentlichen übereinstimmen. Es kann erforderlich sein, dass das Bild der Magnetresonanzanlage 14 mit einer Art von zum Beispiel Portal-Röntgenbild des der Behandlungsvorrichtung 12 oder mit Behandlungsvorrichtungsplanungsbildern koregistriert wird, idealerweise kann dieser Schritt beispielsweise durch einen automatischen Algorithmus erfolgen. Das Atmungssignal wird mit der Behandlungsvorrichtung 12 verbunden. Wenn der Beatmungsgurt während des Transfers nicht vom Patienten 20 abgenommen und die Beatmung zur Überwachungseinheit nicht abgetrennt wurde, ist es beispielsweise auch möglich, in derselben Beatmungsphase weiterzumachen, das heißt das Atmungssignal ist von dem Scan bis zur Behandlung selbst kontinuierlich. Im Idealfall wird der Patient 20 verlegt, ohne dass der Beatmungsgürtel, die Kissen und dergleichen bewegt werden, sodass der Atmungszustand kontinuierlich verfolgt werden kann. Das aus den Scanbildern und dem Atmungssignal abgeleitete Bewegungsmodell 28a, 28b wird in der Behandlungsvorrichtung 12 fortgeführt und liefert eine genaue Vorhersage der Organ- und Zielgebietsposition während der Bestrahlung 22. Daher kann die Patientenbewegung, insbesondere die Atembewegung, des Patienten 20 während der Behandlung 22 auf der Grundlage des Bewegungsmodells 28b der Organe des Patienten 20 unter Verwendung eines relativ einfachen externen Atemsignals vorhergesagt werden.

Die Erfindung kann beispielsweise noch dadurch erweitert werden, dass unterschiedliche Sequenzen in der Magnetresonanzanlage 14 bereitgestellt werden, zum Beispiel Beschleunigungstechniken, Spuren und dergleichen, um ein besseres Bewegungsmodell 28a, 28b und Informationen über die Organe und das Zielgebiet zu erhalten. Des Weiteren kann eine Verwendung verschiedener Signalquellen für das Atmungssignal, zum Beispiel kann das Atmungsband durch induktive oder kapazitive Sensoren, Ultraschallsonden, Ultrabreitbandradar oder dergleichen eingesetzt werden. Das Atmungssignal kann durch andere Signale ergänzt werden, wie zum Beispiel Quellen für die Herzfrequenz oder andere Bewegungssensoren, zum Beispiel eine aus Ultraschall abgeleitete peristaltische Bewegung. Das Atmungssignal kann während der Sitzungen von der kontinuierlichen Überwachung abgekoppelt werden, und die Wiederanbindung, insbesondere die Phase der Atmungskurve, wird zum Beispiel durch ein Röntgenbild ermittelt, das während oder kurz vor der Bestrahlung 22 aufgenommen wurde. Das Atmungssignal während der Bestrahlung 22 kann durch dreidimensionale oder vierdimensionale Formationen ergänzt werden, die durch Röntgenaufnahme oder andere Techniken während der Bestrahlung 22 gewonnen werden. Das Bewegungsmodell 28a, 28b, welches insbesondere dem digitalen Zwilling des Patienten 20 entspricht, kann durch Informationen von zum Beispiel dreidimensionalen Kameras oder dergleichen weiterverbessert werden. Ferner kann die Einhaltung eines bestimmten Atemmusters, idealerweise desjenigen, das während des Scans verwendet wird, durch eine Rückmeldung an den Patienten, insbesondere ein visuelles Feedback über eine Patientenunterhaltungs-/Leitsystem, beispielsweise der Anzeigeeinrichtung 36, gesteuert werden.

Letztendlich kann das Bewegungsmodell 28b der Organe beziehungsweise der digitale Zwilling des Patienten 20 auch verwendet werden, um nicht nur einen Bewegungsausgleich innerhalb einer Sitzung für die Behandlung 22 zu gewährleisten, sondern auch einen Bewegungs-und Organabgleich zwischen den einzelnen Behandlungssitzungen an verschiedenen Tagen zu ermöglichen. Zu diesem Zweck kann entweder der Scan vor jeder Behandlungssitzung wiederholt und die jeweiligen Unterschiede in der Organposition zwischen den von verschiedenen Tagen abgeleiteten Bewegungsmodellen 28a, 28b verfolgt und kompensiert werden, oder das Bewegungsmodell 28a, 28b kann als konstant angenommen werden und der Abgleich der Folgesitzungen würde zum Beispiel über das vom RT-Gerät, insbesondere der Behandlungsvorrichtung 12, gewonnenen Atmungssignal und/oder andere Leitlinien in der Behandlungsvorrichtung 12, wie das Röntgensignal während der Sitzung, erfolgen. Das In-Session-Röntgensignal der Behandlungsvorrichtung 12 kann auch verwendet werden, um die für das Bewegungsmodell 28a, 28b verwendeten Bilddaten und den Koordinatenrahmen abzugleichen, wenn die Übertragung, beispielsweise auf der Trage 26, nicht perfekt ist.

Darüber hinaus kann das Bewegungsmodell 28a, 28b beziehungsweise der digitale Zwilling nicht nur zum Ein- und Ausschalten der Bestrahlung 22 verwendet werden, um eine Bewegung zu kompensieren, sondern auch als zusätzlicher Eingangsparameter für die Modulation der Intensität, zum Beispiel durch Berechnung von Dämpfungskarten, synthetischen CT-Karten aus dem Bewegungsmodell 28a, 28b, realisiert werden.

Das vorgestellte Verfahren hat insbesondere die Vorteile, dass das Bewegungsmodell 28a, 28b auf dem individuellen Patienten basiert und berücksichtigt die entsprechenden Organbewegungen im Gegensatz zu externen Signalen. Es kann ein Weichteilkontrast des MR-Signals verwendet werden, der in der Regel eine hervorragende Identifizierung des Zielvolumens und der Organe ermöglicht. Es ist dabei kein gleichzeitiges MR-Linac-System erforderlich, das technisch komplex, schwierig für die Patientenpositionierung und teuer ist. Die Technik kann auf eine Vielzahl von bereits installierten Behandlungsvorrichtungen 12 und Magnetresonanzanlagen 14 angewendet werden, das heißt sie kann an eine installierte Basis genutzt werden. Ferner kann ein hervorragender Weichteilkontrast realisiert werden und die Fähigkeit, Bewegungsmodelle 28a, 28b zu konstruieren und abzuleiten, wobei dies mit vorhandener Behandlungsvorrichtungshardware kombiniert werden kann oder erfordert nur eine minimale Hardwareänderung. Ferner kann eine Vielzahl von unterschiedlichen Magnetresonanzanlagen 14 eingesetzt werden. Außer der Verfügbarkeit der entsprechenden Bildgebungssequenz und des Triggersignaleingangs sind keine besonderen Anforderungen an die Magnetresonanzanlage 14 erforderlich. Die Magnetresonanzanlage 14 muss sich dabei nicht im selben Raum oder sogar in derselben Abteilung wie die Bestrahlungsvorrichtung 12 befinden, ist jedoch bevorzugt vorgesehen. In einer ersten Ausführungsform kann ein einfacher Atmungsgürtel ausreichen, wodurch der Aufwand für komplexere Erkennungsmechanismen an der Behandlungsvorrichtung 12 minimiert ist. Die Anwendbarkeit und Übersetzung einer bereits bestehenden Lösung für die PET/MR-Bildgebung minimiert einen Entwicklungsaufwand und ermöglicht eine schnelle Implementierung und Prüfung. Im Gegensatz zur eigenständigen RT-Lösung für die entsprechende Bildgebung mit der Magnetresonanzanlage 14, die Bewegungsparameter zur Optimierung der Bildqualität anwenden, die später für die Planung der Bestrahlung 22 verwendet wird, konzentriert sich dieser Ansatz auf die Bewegungskorrektur während der Behandlung.

## Patentansprüche

1. Verfahren zum Erzeugen von Steuerdaten (18) für eine Bestrahlungsvorrichtung (12) für einen Patienten (20) mittels einer elektronischen Recheneinrichtung (16) der Bestrahlungsvorrichtung (12), mit den Schritten:
- Bereitstellen eines Bewegungsmodells (28a, 28b) des Patienten (20) für eine Bestrahlung (22) in der Bestrahlungsvorrichtung (12); und
- Erzeugen von Steuerdaten (18) für die Bestrahlung (22) des Patienten (20) in Abhängigkeit von zumindest einer Patienteninformation und in Abhängigkeit von dem Bewegungsmodell (28a, 28b).

2. Verfahren nach Anspruch 1, wobei das Bewegungsmodell (28a, 28b) auf Basis einer außerhalb der Bestrahlungsvorrichtung (12) erfassten Bewegung des Patienten (20) zeitlich vor der Bestrahlung (22) bestimmt wird.

3. Verfahren nach Anspruch 2, wobei auf Basis einer mittels einer Magnetresonanzanlage (14) bestimmten Bewegung des Patienten (20) in der Magnetresonanzanlage (14) das Bewegungsmodell (28a, 28b) erzeugt wird.

4. Verfahren nach Anspruch 3, wobei eine Position (P) des Patienten (20) in der Magnetresonanzanlage (14) und eine Position (P) des Patienten (20) in der Bestrahlungsvorrichtung (12) zumindest im Wesentlichen gleich eingestellt werden.

5. Verfahren nach einem der vorhergehenden Ansprüche, wobei eine Atmung als die Bewegung des Patienten (20) im Bewegungsmodell (28a, 28b) berücksichtigt wird.

6. Verfahren nach einem der vorhergehenden Ansprüche, wobei eine Bewegung von Organen des Patienten (20) mittels des Bewegungsmodells (28a, 28b) vorhergesagt wird.

7. Verfahren nach einem der vorhergehenden Ansprüche, wobei mittels der Steuerdaten (18) eine Intensität der Bestrahlung (22) und/oder eine Position der Bestrahlung (22) und/oder ein Zeitverlauf der Bestrahlung (22) eingestellt werden.

8. Verfahren nach einem der vorhergehenden Ansprüche, wobei eine Bestrahlungszone für die Bestrahlung (22) vorgeben wird und das Bewegungsmodell (28a, 28b) zumindest für die Bestrahlungszone mittels der elektronischen Recheneinrichtung (16) erzeugt wird.

9. Verfahren nach einem der vorhergehenden Ansprüche, wobei während einer Bestrahlung (22) des Patienten eine aktuelle Atmung (32) des Patienten (20) erfasst wird und auf Basis der aktuellen Atmung (32) die Steuerdaten (18) erzeugt werden.

10. Verfahren nach Anspruch 9, wobei die aktuelle Atmung (32) mittels eines Atemgurtes und/oder mittels einer Kamera während der Bestrahlung (22) erfasst wird.

11. Verfahren nach einem der vorhergehenden Ansprüche, wobei ein Atemsignal (36) für den Patienten (20) während der Bestrahlung (22) zum Nachatmen vorgegeben wird.

12. Computerprogrammprodukt mit Programmcodemitteln, welche eine elektronische Recheneinrichtung (16) dazu veranlassen, wenn die Programmcodemittel von der elektronischen Recheneinrichtung (16) abgearbeitet werden, ein Verfahren nach einem der Ansprüche 1 bis 11 durchzuführen.

13. Computerlesbares Speichermedium mit zumindest dem Computerprogrammprodukt nach Anspruch 12.

14. Elektronische Recheneinrichtung (16) zum Erzeugen von Steuerdaten (18) für eine Bestrahlungsvorrichtung (12) für einen Patienten (20), wobei die elektronische Recheneinrichtung (16) zum Durchführen eines Verfahrens nach einem der Ansprüche 1 bis 11 ausgebildet ist.

15. Bestrahlungsvorrichtung (12) zum Bestrahlen von einem Patienten (20), mit zumindest einer elektronischen Recheneinrichtung (16) nach Anspruch 14.
